(19) Europäisches Patentamt
European Patent Office
Office européen des brevets

(11) EP 1 736 463 B1

(12) # EUROPEAN PATENT SPECIFICATION

(45) Date of publication and mention of the grant of the patent:
**05.10.2011 Bulletin 2011/40**

(51) Int Cl.:
***C07C 209/50*** *(2006.01)* ***C07C 211/08*** *(2006.01)*

(21) Application number: **05105524.2**

(22) Date of filing: **22.06.2005**

(54) **Process for obtaining amines by reduction of amides**

Verfahren zur Herstellung von Aminen durch Reduktion von Amiden

procede de preparation d'amines par reduction d'amides

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **21.06.2005 PCT/EP2005/052886**

(43) Date of publication of application:
**27.12.2006 Bulletin 2006/52**

(73) Proprietor: **Taminco**
**9000 Gent (BE)**

(72) Inventors:
- **Loenders, Raf**
**3360 Bierbeek (BE)**
- **Vanden Eynde, Ivan**
**3140 Keerbergen (BE)**
- **Vanneste, Piet**
**9400 Denderwindeke (BE)**

(74) Representative: **Van Reet, Joseph et al**
**Gevers**
**Intellectual Property House**
**Holidaystraat 5**
**1831 Diegem (BE)**

(56) References cited:
**US-A- 2 166 971**
**US-A- 2 187 745**
**US-A- 3 190 922**
**US-A- 5 075 505**
**US-A- 5 840 985**

EP 1 736 463 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Background of the invention

The present invention relates to an improved process for obtaining amines with the following formula

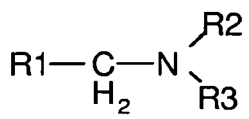

by catalytic reduction of the corresponding amide. The amines should be obtained with high yield and purity. They can be used in a broad range of applications including agrochemicals, pharmaceuticals, detergents, personal care.

Prior art

There are several well described processes for obtaining such amines. Aldehydes, ketons or alcohols react with ammonia or amines under reductive conditions to form primary, secondary or tertiary amines. Another route starts from nitriles, which are reduced to primary amines and if necessary transformed to tertiary amines by reaction with formaldehyde. For speciality products, like pharmaceutical intermediates, the starting material is often an organic halogenide, which reacts readily with ammonia or an amine to form an organic amine salt. High value amine products can also be obtained by reducing amides with powerful reducing agents like $LiBH_4$ or $AlH_3$.

However, when using catalytic hydrogenation the amide reduction route becomes attractive for producing a broad range of amines, especially for bulk processes. For the production of fatty dimethylamines for instance, the fatty amide can be easily obtained by reacting the fatty acid with dimethylamine. By reducing this amide the desired amine is obtained. This route offers an economically interesting alternative for the fatty alcohol or fatty nitrile route (currently used to produce fatty amines). Patents describing the catalytic hydrogenation of fatty amides have been published since the early 60s (see for example US-A-3 190 922, US-A-3 444 204). Until now, however, processes describing the reduction of amides are not very appealing due to low activity and/or side product formation; especially fatty alcohol and dialkylamines (containing a further R1-CH2 group instead of an R2 or an R3 group).

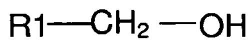

fatty alcohol

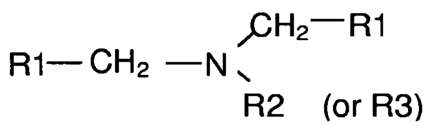

Dialkylamine

The dialkylamine side products will increase the distillation residue while fatty alcohols, due to little difference in boiling temperature, cannot be separated from the fatty amine and thus reduce the product quality.

In the prior art the catalytic hydrogenation of fatty amides has been performed either in a liquid or in a gaseous phase.

When working in slurry, with recycle of hydrogen, the reaction is usually carried out under a relatively low reaction pressure, i.e. under reaction pressures lower than 55 bars. A number of patent publications which describe a liquid phase slurry process to produce an alkyldimethyl amine, namely US-A-3 190 922, US-A-5 840 985 and US-A-5 075 505, teach to add DMA (dimethylamine) in order to reduce the formation of fatty alcohol. However, it appears for example from comparative example 1 of US-A-5 840 985, from example 2 of US-A-5 075 505 and from example 5 of US-A-3 190 922 that although the addition of DMA contributes to solving the problem of the alcohol formation it does not help to solve the problem of the dialkylamine side products which are formed. On the contrary, when comparing example 5 of US-A-3 190 922 with example 1 thereof, it even appears that the addition of DMA increases the formation of these dialkylamine side products.

US-A-5 840 985 discloses to solve the problem of the dialkylamine side product formation by addition of sodiummethoxide to the solution. The addition and removal of this salt is of course reflected in the overall economics of the process. Moreover, the scale up of these slurry processes is not straightforward among others due to the difficult catalyst recycle. Especially continuous operation becomes a difficult issue. Moreover, there is a real risk of catalyst (like CuCr, Ni,...) ending up in the product.

The known fixed bed processes wherein the amide is at least partially led in the gaseous phase over the catalyst can also not compete with the fatty alcohol or nitrile processes. Pashkova,L.P.; Yakashkin,M.I. Zhurnal Prikladnoi Khimii, Vol.53, No.8, pp 1834-1837 disclose a process wherein the hydrogenation reaction is carried out at atmospheric pressure. Hydrogen is supplied to the reactor at a molar hydrogen/amide ratio of 10/1. Notwithstanding the use of such a relatively high amount of hydrogen and notwithstanding the fact that in the examples the amide was only led over the catalyst at a flow rate of 0.16 g/g catalyst/hour, not only selectivity but also conversion is low. The reaction product contained, apart from a small amount of alcohol, a large amount of other side products.

In order to increase the conversion, the hydrogenation reaction can be performed under higher reaction pressures as disclosed for example in US-A-3 444 204. This US patent discloses a pressure range of between 50 and 300 atmospheres but all of the examples are performed at a pressure of 250 or 260 atmospheres At such high pressures activity is reasonably good, but from the examples of US-A-3 444 204 it appears that the selectivity or yield is still low due to the formation of the undesired dialkylamines (see for example Example 1 of US-A-3 444 204; ca. 4.5% of dialkylmethylamine is formed) and, in case of more realistic catalyst contact times, also due to the formation of alcohols (see for example Example 2 of US-A-3 444 204). A further important drawback of the hydrogenation process disclosed in US-A-3 444 204 is that the use of the high reaction pressures implicate high construction and operation costs. In the process disclosed in US-A-3 444 204 the high reaction pressure appears however to be essential to achieve the best possible yield, although this yield is still too low.

According to the present inventors the fact that the process disclosed in US-A-3 444 204 requires high reaction pressures to optimise the yield can be explained as follows. When a film of liquid is formed onto the surface of the catalyst a high pressure is required to achieve a sufficient hydrogen concentration on the surface of the catalyst. As appears for example from c. 4, l. 16-20 of US-A-5 075 505 hydrogen diffuses indeed badly into the amide. Such a diffusion is however required in the process disclosed in US-A-3 444 204 since, under the high pressures used in that process, a large portion of the amide will still be in liquid form notwithstanding the fact that a lot of hydrogen is used. In fact in examples 1 and 2 only about 20wt% of the amide will be in the vapour form due to the low vapour pressure of the amide combined with the high pressure in the reactor. Consequently, a film of liquid amide and reaction products will be present on the surface of the catalyst wherein the hydrogen has to diffuse to be able to react. This diffusion is enhanced by the use of higher hydrogen pressures so that, in the process described in US-A-3 444 204, the use of pressures higher than 50 atmospheres is essential to achieve a good conversion.

An object of the present invention is to provide a process for preparing amines in the gaseous phase by catalytic reduction of an amide which enables to achieve the amines with higher yield and purity. The amine corresponds to the following formula:

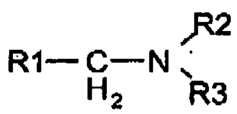,

wherein R1 is H or a saturated or unsaturated hydrocarbon group containing from 1 to 23 carbon atoms and wherein R2 and R3 are independently H or a hydrocarbon group containing from 1 to 8 carbon atoms, and preferably 1 to 4 carbon atoms, and the amine is obtained by reducing an amide of the following formula:

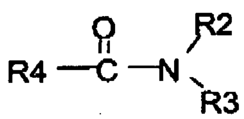,

wherein R4 is equal to R1 but may show a different degree of unsaturation, with hydrogen by means of a hydrogenation catalyst.

Summary of the invention

To achieve the object of the invention, the process is characterised in that the amide is reduced by means of hydrogen in the presence of an auxiliary amine of the following formula:

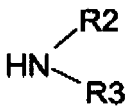,

and in that the hydrogen, the auxiliary amine and the amide are all led completely in a gaseous form, in a gaseous stream, over the hydrogenation catalyst at a reaction pressure higher than 2 but lower than 50 bars.

It was found that when working entirely or at least substantially entirely in the gaseous phase at reasonable low pressures (2-50 bar) and adding an auxiliary amine, high activity and very high selectivity towards the desired amine can be obtained using typical hydrogenation catalysts. At pressures of between 2 and 50 bars, it is economically feasible to use the amount of hydrogen (optionally in combination with an additional carrier gas) which is required to vaporise all of the amide in the feedstream. At the pressures used in the examples of US-A-3 444 204 it is on the contrary almost not feasible to use an amount of hydrogen which would vaporise all of the amide. The present inventors have done tests showing indeed that the saturated vapour pressure of dimethyllaurylamide at 250°C comprises about 220 mbar. At 250 bar, more than 1000 mol $H_2$/mol dimethyllaurylamide would thus be required for complete amide vaporisation. In the examples given in US-A-3 444 204 much less hydrogen is however used, more particularly such an amount that only a minor part of the amide is actually in the vapour form.

Surprisingly, in the process according to the invention the lower reaction pressure does not result in lower activity. On the contrary, even higher activity was obtained. An important advantage of the relatively low pressures applied in the process according to the invention, is indeed that they enable to use the amount of hydrogen (optionally in combination with other carrier gases) required to vaporise all of the amide. In this way no liquid film is formed on the catalyst so that no high reaction pressure is required to allow the hydrogen to reach the catalyst surface. The combination of a relatively low pressure, a sufficiently high amount of hydrogen and optionally other gases to vaporise all of the reactants and the use of an auxiliary amine was thus found quite surprisingly to enable to obtain a high activity and a very high selectivity towards the desired amine using typical hydrogenation catalysts. The addition of the auxiliary amine did not only reduce the alcohol content in the product, but surprisingly also the formation of the undesired dialkylamines. The low dialkylamine formation is not only important to obtain high yields of the desired amine product, but in case of gasphase operation the high boiling dialkylamines could form a liquid film around the catalyst, blocking the active sites and reducing its activity and life time. This is thus prevented in the process according to the invention by the addition of the auxiliary amine.

In the process according to the invention, the gaseous stream which is led over the catalyst is composed to contain, per mole of said amide, in total at least $N_c$ moles of carrier gasses which comprise at least said auxiliary amine and said $H_2$; with

$$N_c = \frac{P_{tot} - VP_a}{VP_a}$$

wherein:

$P_{tot}$ = the reaction pressure; and

$VP_a$ = the saturated vapour pressure of the amide at the reaction temperature.

When using such an amount of carrier gases, the amide is led entirely in vapour form over the catalyst. Indeed, with such an amount of carrier gasses, the amide is completely vaporised since the partial pressure of the amide is lower than the saturated vapour pressure of the amide at the reaction temperature.

Depending on the starting amide and auxiliary amine, the reaction conditions may differ in temperature, pressure, $H_2$ flow and catalyst.

In practice the carrier gas consists predominantly out of $H_2$ with small quantities of auxiliary amine. Mixtures of $H_2$ and inert gasses like $N_2$ or He are also possible.

By operating like this, a very pure amine product can be obtained, containing no or low unreacted amide and alcohol and only minor amounts of mixed amines by-products. Depending on the application, the obtained product can be used as such or requires a simple purification.

On top of that, the catalyst does not suffer from rapid deactivation. The free access of $H_2$ on the catalysts, protecting it from poisons in the feed or poisonous reaction intermediates, may explain this prolonged life compared to liquid and trickling phase reactions.

The process can be easily operated as a fixed bed process. This offers important advantages over slurry processes, such as a straightforward continuous operation, simple process follow up and no catalyst ending up in the product. The unreacted $H_2$ and auxiliary amines can be easily separated from the product and recycled

The low operation pressure of this invention, compared to the known fixed bed processes, reduces significantly the construction costs and moreover lowers drastically the extremely high $H_2$ excess or additional inert carrier gas (and thus recycle stream) necessary to vaporise the high boiling amides.

Detailed description of the invention

The present invention relates to a process for the production of primary, secondary or tertiary amines of following formula:

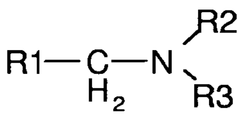

wherein R1 is H or a saturated or unsaturated hydrocarbon group containing from 1 to 23 carbon atoms and wherein R2 and R3 are independently H or a hydrocarbon group containing from 1 to 8 carbon atoms.

The process comprises the reduction with $H_2$ of an amide of following formula:

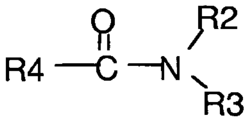

wherein this amide is led together with an auxiliary amine of following formula:

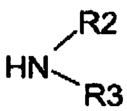,

in a gaseous stream which contains the $H_2$, over a hydrogenation catalyst. In the formula of said amide, R4 is equal to R1 but may show a different degree of saturation. Notice that, in case R2 and R3 are both H, the auxiliary amine is in fact ammonia which has in the present specification also to be understood as being an amine.

An essential feature of the process according to the invention is that the entire amount of the amide which is led over the catalyst is led in gaseous form over the catalyst. This is achieved by using a sufficient amount of hydrogen, optionally in combination with one or more additional carrier gasses.

The reaction is carried out at a relatively low pressures in the range of between 2 and 50 bars. In view of obtaining a still higher activity, the reaction pressure is preferably higher or equal to 3 bars, more preferably higher or equal to 4 bars and most preferably higher or equal to 5 bars. Since the required amount of carrier gas increases considerably with higher pressures, the reaction pressure is preferably lower than 45 bars, more preferably lower than 30 bars and most preferably lower than 20 bars.

The amount of carrier gases is selected in such a manner that all of the reactants are entirely vaporised. This is achieved when use is made, per mole of amide, of a total at least $N_c$ moles of carrier gasses which comprises the hydrogen, the less important amount of auxiliary amine and any further substances (carrier gasses) which are gaseous under the reaction conditions, with

$$N_c = \frac{P_{tot} - VP_a}{VP_a}$$

wherein:

$P_{tot}$ = the total reaction pressure; and
$VP_a$ = the saturated vapour pressure of the amide at the reaction temperature.

For an amide having a saturated vapour pressure of about 220 mbar at the reaction temperature, $N_c$ is for example equal to:

22 for a reaction pressure of 5 bars;
44 for a reaction pressure of 10 bars;
90 for a reaction pressure of 20 bars;
226 for a reaction pressure of 50 bars (which pressure is not in accordance with the present invention); and
1135 for a reaction pressure of 250 bars (which pressure is also not in accordance with the present invention).

Typical reaction temperatures range from 100 to 350 °C and $H_2$ dilution varies from 5 to 500 mole/mole amide, all depending on the volatility of the reagents and products as well as on the working pressure and catalyst type.

The reaction is catalysed by typical hydrogenation catalysts. These are usually metal based like Cu, Cr, Ni, Co, Pd, Pt, Ru or mixtures thereof, optionally on support and in the presence of modifiers like Li, Na, K, Ba, Mg, Ca, Mn, Zr. The most preferred catalyst is a CuCr-type of catalyst.

The addition of the auxiliary amine strongly reduces the side product formation, in particular alcohol but surprisingly also dialkylamine, when the reaction is performed entirely in the gaseous phase as in the process according to the present invention. In a preferred embodiment of the present invention the auxiliary amine / amide mole ratio varies from 0.05 to 40, and is preferably higher than 0.4, more preferably higher than 0.6. In a preferred embodiment, the auxiliary amine / amide mole ratio is lower than 30, preferably lower than 15, and most preferably lower than 5. The lower the auxiliary amine/amide ratio, the higher the side product formation while higher ratios are not desired for reasons of capacity loss and large amine recycle streams. Due to absence of consumption, the auxiliary amine can be recycled. Also addition of large quantities of tertiary auxiliary amines ($(R2)_3N$) do not effect the selectivity, on the contrary and surprisingly, an improvement of the selectivity could be observed in cases where R2 and R3 are the same and consist of a hydrocarbon group with 1 to 4 carbon atoms.

In view of the high selectivity and activity of the process according to the invention, the amide and other reactants can be led at a relatively high flow rate over the catalyst, notwithstanding the fact that the amide is quite diluted in the carrier gas. In view of obtaining a high selectivity, especially to avoid alcohol and undesired dialkylamines in the product stream, the amount of catalyst, or in other words the contact time with the catalyst, is preferably sufficient to convert more than 95wt%, preferably more than 98wt%, and more preferably more than 99 wt% of the amide. The catalyst is preferably a fixed bed catalyst.

If the desired amine is a tertiary amine with R2 and R3 being the same, any secondary amine present in the product which is separated off from the gaseous stream which has passed over the catalyst can be converted quite easily to the desired tertiary amine by an additional alkylation step.

Example 1

In a fixed bed reactor containing 15 g of CuCr catalyst (Leuna 1970T) a mixture of N,N-dimethyldecylamide, DMA and $H_2$ is introduced. The mixture with a molar composition of amide/auxiliary amine/$H_2$ of 1/3/120 is preheated and introduced continuously at the catalyst bed at a rate of 15 g amide/h. This mixture comprises, per mole amide, 123 moles of carrier gases. The reactor is heated at 250°C and operated at 10 bars. At the outlet, the product containing the amine is separated from the gas by condensation. The liquid outlet is analysed using gaschromatography, the composition (area%) is displayed in table 1. By a simple methylation reaction, N-methyldecylamine can be converted to N,N-dimethyldecylamine reducing the side products to less than 1%.

Table 1: product distribution (GC analysis, area%)

| N,N-dimethyl decylamine | N-methyl decylamine | N-methyl didecylamine | N,N-dimethyl decylamide | Decanol |
|---|---|---|---|---|
| 96.3 | 2.7 | 0.7 | Nd | 0.1 |
| Nd: not detected | | | | |

Example 2

In a fixed bed reactor containing 50 g of CuCr catalyst (Cu0203 Engelhard) a mixture of N,N-dimethyldodecyl amide, DMA and $H_2$ with following molar ratio 1/3/76 is fed continuously at an amide rate of 20 g/h. This mixture comprises, per mole amide, 79 moles of carrier gasses. Prior to the catalyst bed, the mixture passes a pre-heater at 250°C. The catalyst bed is also heated at 250°C and the reactor is operated at 5 bars. Since the saturated vapour pressure of N,N-dimethyldodecyl amide at 250°C comprises about 220 mbar, at least about 22 moles of carrier gasses $(= \frac{5-0.22}{0.22})$ are required to vaporise all the amide so that in this example all of the amide is vaporised. At the outlet, the product containing the amine is separated from the gas flow (containing the $H_2$ and the auxiliary amine) by condensation. The liquid outlet is analysed daily using gaschromatography, the composition (area%) is displayed in table 2. No deactivation is observed after 1 month of testing.

Table 2: product distribution (Gc analysis, area%)

| Day | N,N-dimethyl dodecylamine | N-methyl dodecylamine | N-methyl didodecylamine | N,N-dimethyl dodecylamide | dodecanol |
|---|---|---|---|---|---|
| 1 | 97.6 | 1.8 | 0.6 | Nd | Nd |
| 10 | 97.6 | 1.8 | 0.5 | Nd | Nd |
| 30 | 97.5 | 1.8 | 0.6 | Nd | Nd |
| Nd: not detected | | | | | |

Example 3

In a fixed bed reactor containing 5 g of catalyst (Cu0203 Engelhard) a mixture of N,N-dimethylacetamide, DMA and $H_2$ is introduced. The mixture with a molar composition of amide/auxiliary amine/$H_2$ of 1/3/30 is preheated and introduced continuously at the catalyst bed at a rate of 2 g amide/h. This mixture comprises, per mole amide, 33 moles of carrier gases. The reactor is heated at 250°C and operated at 10 bars. At the outlet, the product containing the desired amine is analysed with GC on-line. The product composition (area%) is displayed in table 3.

Table 3: product distribution (GC analysis, area%)

| N,N-dimethyl ethylamine | N-methyl ethylamine | N-methyl diethylamine | N,N-dimethyl acetamide | ethanol |
|---|---|---|---|---|
| 96.1 | 2.8 | 0.8 | Nd | Nd |
| Nd: not detected | | | | |

Example 4

In a fixed bed reactor containing 5 g of catalyst (Leuna 1970T) a mixture of acetamide, $NH_3$ (as auxiliary amine) and $H_2$ is introduced. The mixture with a molar composition of amide/ammonia/$H_2$ of 1/10/30 is preheated and introduced continuously at the catalyst bed at a rate of 2 g amide/h. This mixture comprises, per mole amide, 40 moles of carrier gases. The reactor is heated at 250°C and operated at 10 bars. At the outlet, the product containing ethylamine is analysed with on-line GC. The product composition (area%) is displayed in table 4

Table 4: product distribution (GC analysis, area%)

| ethylamine | diethylamin e | triethylamine | acetamide | ethanol |
|---|---|---|---|---|
| 95.5 | 3.5 | 0.5 | Nd | Nd |
| Nd: not detected | | | | |

Comparative example A

In the same reactor set up, example 1 is repeated except for the DMA addition. The selectivity towards N,N-dimethyldecylamine is reduced to 92.5 %, mainly at expense of N-methyldidecylamine and decanol.

Table 5: product distribution (Gc analysis, area%)

| N,N-dimethyl decylamine | N-methyl decylamine | N-methyl didecylamine | N,N-dimethyl decylamide | decanol |
|---|---|---|---|---|
| 92.5 | 0.4 | 4.7 | Nd | 2.0 |
| Nd: not detected | | | | |

Comparative example B

In the same reactor set-up as example 2, containing 50 g of CuCr catalyst (Cu0203 of Engelhard) a N,N-dimethyldodecylamide/DMA/$H_2$ mixture with following composition 1/3/8 (molar) was fed continuously at a rate of 5 g amide/h. This mixture comprises, per mole amide, 11 moles of carrier gases. The reaction was carried out at 10 bars and 250°C and the product outlet was analysed daily.

Since the saturated vapour pressure of N,N-dimethyldodecyl amide at 250°C comprises about 220 mbar, at least about 44 moles of carrier gases $\left(= \frac{10 - 0.22}{0.22}\right)$ are required to vaporise all the amide. In this experiment the $H_2$ dilution is thus far too little to vaporise 95% of the reagents, resulting in very poor activity and selectivity. After one week already a catalyst decay is observed.

Table 6: product distribution (Gc analysis, area%)

| Day | N,N-dimethyl dodecylamine | N-methyl dodecylamine | N-methyl didodecylamine | N,N-dimethyl dodecylamide | dodecanol |
|---|---|---|---|---|---|
| 1 | 30.2 | 5.5 | 22.1 | 41.8 | 0.4 |
| 7 | 24.1 | 4.7 | 26.4 | 44.4 | 0.4 |

## Claims

1. A process for preparing an amine with following formula:

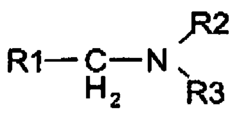

comprising the reduction of an amide of following formula:

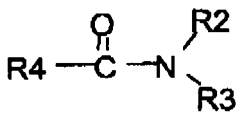

with $H_2$ in the presence of an auxiliary amine of the following formula:

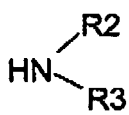

wherein:

R1 is H or a saturated or unsaturated hydrocarbon group containing from 1 to 23 carbon atoms;
R2 and R3 are independently H or a hydrocarbon group containing from 1 to 8 carbon atoms, and preferably 1 to 4 carbon atoms, and
R4 is equal to R1 but may show a different degree of unsaturation,

in which process
said $H_2$, said auxiliary amine and said amide are all led completely in a gaseous form, in a gaseous stream, over a hydrogenation catalyst, at a reaction pressure higher than 2 but lower than 50 bars, and
said gaseous stream is composed to contain, per mole of said amide, at least $N_c$ moles of carrier gases which comprise at least said auxiliary amine and said $H_2$; with

$$N_c = \frac{P_{tot} - VP_a}{VP_a}$$

wherein:

$P_{tot}$ = the reaction pressure; and
$VP_a$ = the saturated vapour pressure of the amide at the reaction temperature.

2. A process according to claim 1, wherein said reduction reaction is performed under a reaction pressure higher than or equal to 3 bars.

3. A process according to claim 2, wherein said reduction reaction is performed under a reaction pressure higher than or equal to 4 bars.

4. A process according to claim 3, wherein said reduction reaction is performed under a reaction pressure higher than or equal to 5 bars.

5. A process according to any one of the claims 1 to 4, **characterised in that** said reduction reaction is performed under a reaction pressure lower than 45 bars.

6. A process according to claim 5, **characterised in that** said reduction reaction is performed under a reaction pressure lower than 30 bars.

7. A process according to claim 6, **characterised in that** said reduction reaction is performed under a reaction pressure lower than 20 bars.

8. A process according to any one of the claims 1 to 7, wherein the auxiliary amine is added in an amount of 0.05 to 40 moles per mole of said amide, preferably in an amount higher than 0.4 moles per mole of said amide, and more preferably in a amount higher than 0.6 moles per mole of said amide

9. A process according to claim 8, **characterised in that** the added amount of auxiliary amine is smaller than 30 moles per mole of said amide, preferably smaller than 15 moles per mole of said amide and more preferably smaller than 5 moles per mole of said amide.

10. A process according to any one of the claims 1 to 9, wherein R2 and R3 are the same and consist of a hydrocarbon group with 1 to 4 carbon atoms and wherein said reduction is carried out in the presence of a further auxiliary amine with following formula:

$$(R2)_3N.$$

11. A process according to any one of the claims 1 to 10, wherein after having led the amide and the auxiliary amine in said gaseous stream over the catalyst, a product containing the amine which is to be prepared is separated off from the gaseous stream.

12. A process according to claim 11, **characterised in that** after having separated the amine which is to be prepared

off from the gaseous stream, at least a portion of the hydrogen and of the auxiliary amine which are still present is the gaseous stream are recycled to the catalyst.

**13.** A process according to claim 11 or 12, wherein the amine which is to be prepared is a tertiary amine with R2 and R3 being the same and wherein said product is subjected to an alkylation reaction to convert any secondary amine present therein into said tertiary amine.

**14.** A process according to any one of the claims 1 to 13, wherein said amide is led over the catalyst with a sufficient contact time to convert more than 95wt% of the said amide, preferably more than 98wt% of the said amide, and more preferably more than 99 wt% of the said amide

**15.** A process according to any one of the claims 1 to 14, wherein said catalyst is a fixed bed catalyst.

**Patentansprüche**

**1.** Ein Verfahren zur Herstellung eines Amins mit folgender Formel:

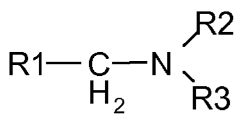

welches die Reduktion eines Amids folgender Formel umfasst:

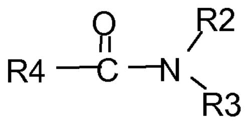

mit $H_2$ in Anwesenheit eines auxiliaren Amins folgender Formel:

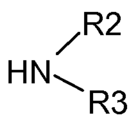

wobei:

R1 H oder eine gesättigte oder ungesättigte Kohlenwasserstoffgruppe ist, die 1 bis 23 Kohlenstoffatome enthält;
R2 und R3 unabhängig H oder eine Kohlenwasserstoffgruppe sind, die 1 bis 8 Kohlenstoffatome, vorzugsweise 1 bis 4 Kohlenstoffatome enthält, und
R4 gleich R1 ist, aber einen anderen Grad an Ungesättigtheit aufweisen kann,

in welchem Verfahren
das erwähnte $H_2$, das erwähnte auxiliare Amin und das erwähnte Amid vollständig in einem gasförmigen Zustand, in einem gasförmigen Strom, über einen Hydrierkatalysator geführt werden, bei einem Reaktionsdruck von mehr als 2 aber weniger als 50 bar, und
der erwähnte gasförmige Strom zusammengesetzt ist, um - pro Mol des erwähnten Amids, mindestens $N_c$ Mole von Trägergasen zu enthalten, die zumindest das erwähnte auxiliare Amin und das erwähnte $H_2$ umfassen; wobei

$$N_c = \frac{P_{tot} - VP_a}{VP_a}$$

wobei:

$P_{tot}$ der Reaktionsdruck ist; und
$VP_a$ = der Sättigungsdampfdruck des Amids bei der Reaktionstemperatur ist.

**2.** Ein Verfahren nach Anspruch 1, in dem die erwähnte Reduktionsreaktion unter einem Reaktionsdruck von mehr als oder gleich 3 bar erfolgt.

**3.** Ein Verfahren nach Anspruch 2, in dem die erwähnte Reduktionsreaktion unter einem Reaktionsdruck von mehr als oder gleich 4 bar erfolgt.

**4.** Ein Verfahren nach Anspruch 3, in dem die erwähnte Reduktionsreaktion unter einem Reaktionsdruck von mehr als oder gleich 5 bar erfolgt.

**5.** Ein Verfahren nach irgendeinem der Ansprüche 1 bis 4, **dadurch gekennzeichnet, dass** die erwähnte Reduktionsreaktion unter einem Reaktionsdruck von weniger als 45 bar erfolgt.

**6.** Ein Verfahren nach Anspruch 5, **dadurch gekennzeichnet, dass** die erwähnte Reduktionsreaktion unter einem Reaktionsdruck von weniger als 30 bar erfolgt.

**7.** Ein Verfahren nach Anspruch 6, **dadurch gekennzeichnet, dass** die erwähnte Reduktionsreaktion unter einem Reaktionsdruck von weniger als 20 bar erfolgt.

**8.** Ein Verfahren nach irgendeinem der Ansprüche 1 bis 7, in dem das auxiliare Amin in einer Menge von 0,05 bis 40 Mol pro Mol des erwähnten Amids zugesetzt wird, vorzugsweise in einer Menge von mehr als 0,4 Mol pro Mol des erwähnten Amids, und noch besser in einer Menge von mehr als 0,6 Mol pro Mol des erwähnten Amids.

**9.** Ein Verfahren nach Anspruch 8, **dadurch gekennzeichnet, dass** die zugesetzte Menge des auxiliaren Amins geringer ist als 30 Mol pro Mol des erwähnten Amids, vorzugsweise geringer als 15 Mol pro Mol des erwähnten Amids und noch besser geringer als 5 Mol pro Mol des erwähnten Amids.

**10.** Ein Verfahren nach irgendeinem der Ansprüche 1 bis 9, in dem R2 und R3 gleich sind und aus einer Kohlenwasserstoffgruppe mit 1 bis 4 Kohlenstoffatomen bestehen und in dem die erwähnte Reduktion in Anwesenheit eines weiteren auxiliaren Amins mit folgender Formel erfolgt:

$$(R2)_3N.$$

**11.** Ein Verfahren nach irgendeinem der Ansprüche 1 bis 10, in dem, nachdem das Amid und das auxiliare Amin im erwähnten gasförmigen Strom über den Katalysator geführt wurden, ein Produkt, welches das Amin enthält, das hergestellt werden soll, aus dem gasförmigen Strom abgeschieden wird.

**12.** Ein Verfahren nach Anspruch 11, **dadurch gekennzeichnet, dass**, nachdem das Amin, das hergestellt werden soll, aus dem gasförmigen Strom abgeschieden wurde, zumindest ein Anteil des Wasserstoffs und des auxiliaren Amins, die noch stets im gasförmigen Strom vorhanden sind, zum Katalysator rückgeführt wird.

**13.** Ein Verfahren nach Anspruch 11 oder 12, in dem das Amin, das hergestellt werden soll, ein tertiäres Amin ist, wobei R2 und R3 gleich sind und wobei das erwähnte Produkt einer Alkylierungsreaktion unterworfen wird, um ein eventuell darin vorhandenes sekundäres Amin in das erwähnte tertiäre Amin umzuwandeln.

**14.** Ein Verfahren nach irgendeinem der Ansprüche 1 bis 13, in dem das erwähnte Amid mit einer ausreichenden Kontaktzeit über den Katalysator geführt wird, um mehr als 95 Gew.-% des erwähnten Amids, vorzugsweise mehr als 98 Gew.-% des erwähnten Amids und noch besser mehr als 99 Gew.-% des erwähnten Amids umzuwandeln.

**15.** Ein Verfahren nach irgendeinem der Ansprüche 1 bis 14, in dem der erwähnte Katalysator ein Festbettkatalysator ist.

## Revendications

**1.** Procédé pour préparer une amine avec la formule suivante :

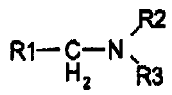

comprenant la réduction d'un amide de la formule suivante :

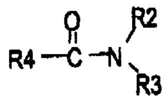

avec $H_2$ en présence d'une amine auxiliaire de la formule suivante :

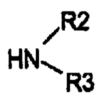

dans laquelle
R1 est H ou un groupe hydrocarbure saturé ou insaturé contenant de 1 à 23 atomes de carbone ;
R2 et R3 sont indépendamment H ou un groupe hydrocarbure contenant de 1 à 8 atomes de carbone, et de préférence de 1 à 4 atomes de carbone, et
R4 est égal à R1 mais peut présenter un degré différent d'insaturation,
dans lequel procédé
ledit $H_2$, ladite amine auxiliaire et ledit amide sont tous amenés totalement dans une forme gazeuse, dans un courant gazeux, au-dessus d'un catalyseur d'hydrogénation, à une pression de réaction supérieure à 2 mais inférieure à 50 bar, et
ledit courant gazeux est composé pour contenir, par mole dudit amide, au moins $N_c$ moles de gaz porteur qui comprend au moins ladite amine auxiliaire et ledit $H_2$; avec

$$N_c = \frac{P_{tot} - VP_a}{VP_a}$$

où :

$P_{tot}$ = la pression de réaction ; et
$VP_a$ = la pression de vapeur saturée de l'amide à la température de réaction.

**2.** Procédé selon la revendication 1, dans lequel ladite réaction de réduction est effectuée sous une pression de réaction supérieure ou égale à 3 bar.

**3.** Procédé selon la revendication 2, dans lequel ladite réaction de réduction est effectuée sous une pression de réaction supérieure ou égale à 4 bar.

**4.** Procédé selon la revendication 3, dans lequel ladite réaction de réduction est effectuée sous une pression de réaction supérieure ou égale à 5 bar.

**5.** Procédé selon l'une quelconque des revendications 1 à 4, **caractérisé en ce que** ladite réaction de réduction est

effectuée sous une pression de réaction inférieure à 45 bar.

6. Procédé selon la revendication 5, **caractérisé en ce que** ladite réaction de réduction est effectuée sous une pression de réaction inférieure à 30 bar.

7. Procédé selon la revendication 6, **caractérisé en ce que** ladite réaction de réduction est effectuée sous une pression de réaction inférieure à 20 bar.

8. Procédé selon l'une quelconque des revendications 1 à 7, dans lequel l'amine auxiliaire est ajoutée en une quantité de 0,05 à 40 moles par mole dudit amide, de préférence en une quantité supérieure à 0,4 mole par mole dudit amide, et mieux encore en une quantité supérieure à 0,6 mole par mole dudit amide.

9. Procédé selon la revendication 8, **caractérisé en ce que** la quantité ajoutée d'amine auxiliaire est inférieure à 30 moles par mole dudit amide, de préférence inférieure à 15 moles par mole dudit amide et mieux encore inférieure à 5 moles par mole dudit amide.

10. Procédé selon l'une quelconque des revendications 1 à 9, dans lequel R2 et R3 sont identiques et consistent en un groupe hydrocarbure comprenant de 1 à 4 atomes de carbone et dans lequel ladite réduction est effectuée en présence d'une autre amine auxiliaire avec la formule suivante :

$$(R2)_3N.$$

11. Procédé selon l'une quelconque des revendications 1 à 10, dans lequel après avoir amené l'amide et l'amine auxiliaire dans ledit courant gazeux au-dessus du catalyseur, un produit contenant l'amine qui doit être préparée est séparé du courant gazeux.

12. Procédé selon la revendication 11, **caractérisé en ce qu'**après avoir séparé l'amine qui doit être préparée du courant gazeux, au moins une partie de l'hydrogène et de l'amine auxiliaire qui sont encore présents dans le courant gazeux sont ramenés vers le catalyseur.

13. Procédé selon la revendication 11 ou 12, dans lequel l'amine qui doit être préparée est une amine tertiaire avec R2 et R3 qui sont identiques et dans lequel ledit produit est soumis à une réaction d'alkylation pour convertir toute amine secondaire présente dans celui-ci en ladite amine tertiaire.

14. Procédé selon l'une quelconque des revendications 1 à 13, dans lequel ledit amide est amené au-dessus du catalyseur avec un temps de contact suffisant pour convertir pour de 95 % en poids dudit amide, de préférence plus de 98 % en poids dudit amide, et mieux encore plus de 99 % en poids dudit amide.

15. Procédé selon l'une quelconque des revendications 1 à 14, dans lequel ledit catalyseur est un catalyseur en lit fixe.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3190922 A
- US 3444204 A
- US 5840985 A
- US 5075505 A


### Non-patent literature cited in the description


- **Pashkova,L.P. ; Yakashkin,M.I.** *Zhurnal Prikladnoi Khimii,* vol. 53 (8), 1834-1837